# EUROPÄISCHE PATENTSCHRIFT

(11) **EP 0 365 971 B1**
(45) Veröffentlichungstag und Bekanntmachung des Hinweises auf die Patenterteilung: **23.02.1994**
(21) Anmeldenummer: 89119204.9
(22) Anmeldetag: 17.10.1989
(51) Int. Cl.: C07C 2/86, C07C 15/16

(54) **Verfahren zur Herstellung von gegebenenfalls substituierten Benzyl-benzolen**
Method for the production of (substituted) benzyl-benzenes
Procédé pour la fabrication de benzènes benzyliques (substitués)

(30) Priorität: 28.10.1988 DE 3836780
(43) Veröffentlichungstag der Anmeldung: 02.05.1990
(73) Patentinhaber: BAYER AG, 51368 Leverkusen (DE)
(72) Erfinder: Klinkmann, Kurt, D-5376 Nettersheim (DE); Herzhoff, Michael, Dr., D-5203 Much (DE); Burmeister, Gerhard, Dr., D-5090 Leverkusen 1 (DE)

(56) Entgegenhaltungen:
- DE-A- 2 039 123
- DE-A- 2 547 030
- DE-C- 638 756

## Beschreibung

Die Erfindung betrifft ein Verfahren zur Herstellung von gegebenenfalls substituierten Benzyl-benzolen durch Umsetzung von gegebenenfalls substituierten Benzylalkoholen oder den daraus hervorgegangenen Dibenzylethern mit gegebenenfalls substituierten Benzolen in Gegenwart von aktivierter Bleicherde bei erhöhter Temperatur.

Gegebenenfalls substituierte Benzyl-benzole sind beispielsweise Diphenylmethan, Benzyl-toluol, Dibenzyl-benzole, Dibenzyl-toluole, Benzyl-biphenyl u.a. Die genannten Verbindungen sind dem Fachmann bekannt.

Benzyl-benzole der genannten Art können nach bekannten Verfahren durch Umsetzung von geeigneten Aromaten mit Formaldehyd hergestellt werden. Nun ist in der letzten Zeit, abgesehen von den toxischen Eigenschaften vieler zur Verwendung kommender Aromaten, die maximale Arbeitsplatzkonzentration (MAK) für Formaldehyd sehr stark abgesenkt worden. Dies bedeutet hohe Aufwendungen für die Arbeitssicherheit und für die wirksame Unterdrückung jeglicher Emission in die Luft oder das Abwasser. Hierdurch wird eine solche Formaldehyd-Chemie in prohibitiver Weise verteuert.

In weiteren ebenfalls bekannten Verfahren können geeignete Aromaten mit Benzylchlorid, beispielsweise in Gegenwart von Friedel-Crafts-Katalysatoren, benzyliert werden. Sowohl die dabei verwendeten Katalysatoren als auch ein restlicher Chlorgehalt oder abgespaltenes Chlor des Benzylchlorids bringen jedoch große Korrosionsprobleme für die in solchen Verfahren verwendeten Apparaturen. Die entstehenden benzylierten Produkte behalten einen Restchlorgehalt, der sie thermisch instabil und eine Verwendung beispielsweise als Wärmeträgeröl daher problematisch macht.

Ferner entsteht bei der Herstellung von Benzylchlorid durch Seitenkettenchlorierung von Toluol stets nebenbei Benzalchlorid. Benzylchlorid und Benzalchlorid können jedoch wegen der Korrosionsprobleme nur in teuren Nickel-Kolonnen destillativ getrennt werden.

Prinzipiell sollte es möglich sein, das Benzylchlorid durch Benzylalkohol zum Zwecke der Benzylierung zu ersetzen. Ein solcher Ersatz hätte wesentliche Vorteile: Zum ersten brächte Benzylalkohol nicht die gefürchtete Chloridionen-Korrosion in ein solches Verfahren; eine bei der Herstellung des Benzylalkohols aus Benzylchlorid auftretende Chloridionen-Korrosion verbliebe jedenfalls im Herstellungsverfahren von Benzylalkohol bzw. Benzylchlorid, wo sie immer bewältigt werden muß, wenn für andere Zwecke als die der vorliegenden Erfindung Benzylchlorid bzw. Benzylalkohol hergestellt werden soll. Zweitens wäre die Abtrennung des bei der Benzylalkohol-Herstellung aus Benzylchlorid stets (aus unvermeidlichem Benzalchlorid) mitentstehenden Benzaldehyds in einer normalen stählernen Destillationskolonne (anstelle der obengenannten Nickel-Kolonne) möglich. Daher hat es bereits Versuche gegeben, die Benzylierung mit Benzylalkohol in Gegenwart von Schwefelsäure oder Phosphorsäure durchzuführen. Solche Versuche brachten jedoch außer der neuen Korrosion durch die genannten starken Säuren große Anteile an harzigen Reaktionsprodukten.

Es wurde auch bereits versucht, die genannten starken Mineralsäuren bei der Benzylierung mit Benzylalkohol durch Bleicherde zu ersetzen. Versuche dieser Art sind in großen zeitlichen Abständen bekanntgeworden, ohne bisher wirtschaftlich nutzbar geworden zu sein.

So ist es aus DE-PS 638 756 bekannt, 2 Teile Benzylalkohol mit 4 Teilen Benzol (molares Verhältnis 1:2,77) bei 230°C in einem Autoklaven umzusetzen. Hierzu werden 50 Gew.-% Tonsil, bezogen auf den Benzylalkohol, eingesetzt. In einer Ausbeute von etwa 40 % der theoretischen Ausbeute wird Diphenylmethan hergestellt und in einer Ausbeute von etwa 25 % Dibenzyl-benzole, alle Ausbeuten auf Benzylalkohol bezogen.

Aus J. General Chemistry of the USSR (englische Übersetzung von Z. obsz. Zhim.) 20 (1950), 2249 (im Original Seite 2168) ist eine Benzylierung einiger Aromaten mit Benzylalkohol in Gegenwart von Askanit bekannt. In dieser Vorschrift wird bei 90°C und 10 Gew.-% Askanit, bezogen auf den Benzylalkohol, gearbeitet. Bei Verwendung von 200 g Benzylalkohol und 200 g Benzol (molares Verhältnis 1:1,38) werden etwa 16 % Diphenylmethan und 4,6 % Dibenzyl-benzol, alles bezogen auf Benzylalkohol, erhalten; der nicht destillierbare Rückstand ist größer als die gesamte Menge an Diphenylmethan und Dibenzyl-benzol. Bei der Benzylierung von Toluol unter den gleichen Bedingungen (molares Verhältnis 1:1,17) werden nur 20 % Benzyl-toluol und 10,8 % Dibenzyl-toluol, beides bezogen auf Benzylalkohol, erhalten.

Eine Nacharbeitung beider Literaturstellen ergab neben den geringen Ausbeuten schwarzbraune Reaktionsprodukte, die in dieser Form keiner weiteren Verwendung zugeführt werden können.

Es wurde nun überraschend gefunden, daß wesentlich größere Mengen an Reaktionsprodukten erhalten werden, wenn man mit wesentlich kleineren Mengen an aktivierter Bleicherde in dem weiter unten genannten Temperaturbereich arbeitet und die Reaktionspartner in ausreichender Verdünnung hält, obwohl dies wegen der geringeren Raum-Ausbeute als technisch rückschrittlich angesehen werden mußte. Die Reaktion ist dann so gut zu steuern, daß bezüglich der Verteilung höherer und niederer Molekulargewichte der Reaktionsprodukte sehr gut differenziert werden kann.

Die Erfindung betrifft demnach ein Verfahren zur Herstellung von Benzyl-benzolen der Formel 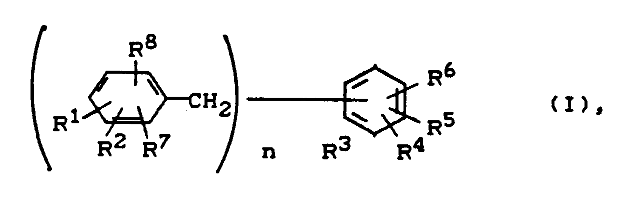
in der
- R¹, R², R³, R⁴ und R⁵: unabhängig voneinander Wasserstoff, C₁-C₄-Alkyl, C₁-C₄-Alkoxy, Fluor oder Chlor bedeuten, wobei R¹, R², R³ und R⁴ unabhängig voneinander auch für Hydroxy stehen können und R⁵ zusätzlich C₅-C₈-Alkyl sein kann,
- R⁶: für Wasserstoff oder eine der Gruppen 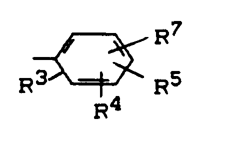 oder 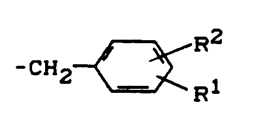 steht,
- R⁷: für Wasserstoff oder die Gruppe 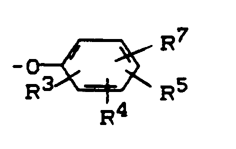 steht und
- n: die Zahl 1 oder 2 darstellt und für den Fall, daß einer der Reste R³, R⁴, R⁵ oder R⁶ Wasserstoff bedeutet, auch die Zahl 3 darstellen kann,
das dadurch gekennzeichnet ist, daß man ein Benzol der Formel 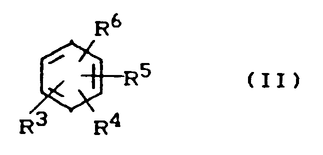
mit einem Benzylalkohol der Formel 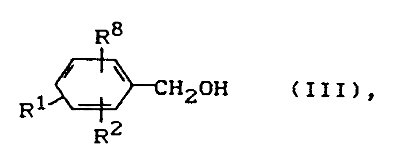
wobei R¹ bis R⁵ die oben angegebene Bedeutung haben,
- R⁶: für Wasserstoff oder eine der Gruppen 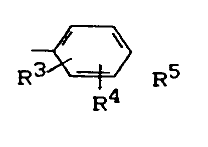 oder 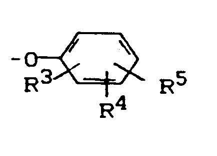 steht und
- R⁸: Wasserstoff oder Hydroxymethyl bedeutet,
in einem Verhältnis von 0,4 bis 15 Mol Benzol zu 1 Mol Benzylalkohol in Gegenwart einer Menge an aktivierter Bleicherde von 0,05-5 Gew.-%, bezogen auf die Menge an Benzylalkohol, bei einer Temperatur zwischen 90°C und 140°C in Gegenwart eines Verdünnungsmittels umsetzt.

C₁-C₈-Alkyl bzw. C₁-C₄-Alkoxy sind beispielsweise Methyl, Ethyl, Propyl, i-Propyl, Butyl, i-Butyl, geradkettiges oder verzweigtes Amyl, Hexyl, Heptyl oder Octyl, Methoxy, Ethoxy, Propoxy, i-Propoxy, Butoxy bzw. i-Butoxy. Bevorzugt ist C₁-C₄ Alkyl, besonders bevorzugt sind Methyl und Ethyl; bevorzugtes Alkoxy sind Methoxy und Ethoxy.

Bevorzugt im erfindungsgemäßen Verfahren einsetzbare Benzylalkohole sind solche der Formel 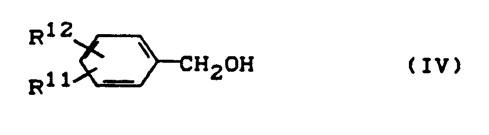
worin
- R¹¹ und R¹²: unabhängig voneinander Wasserstoff, Methyl, Ethyl, Fluor oder Chlor bedeuten und R¹¹ zusätzlich für Hydroxymethyl stehen kann.

Besonders bevorzugte Benzylalkohole für das erfindungsgemäße Verfahren sind solche der Formel 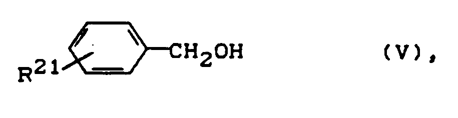
worin
- R²¹: Wasserstoff, Methyl oder Ethyl bedeutet.

In ganz besonders bevorzugter Weise wird der unsubstituierte Benzylalkohol eingesetzt.

Bevorzugte Benzol-Verbindungen für das erfindungsgemäße Verfahren sind solche der Formel 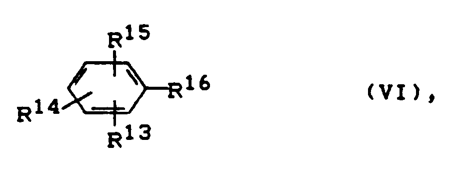
worin
- R¹³, R¹⁴ und R¹⁵: unabhängig voneinander Wasserstoff, C₁-C₄-Alkyl, Fluor oder Chlor bedeuten und R¹³ weiterhin für Hydroxy stehen kann und
- R¹⁶: für Wasserstoff oder eine der Gruppen 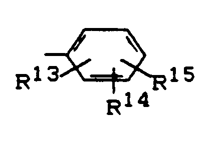 oder 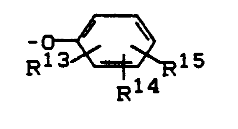 steht.

Besonders bevorzugte Benzol-Verbindungen sind solche der Formel 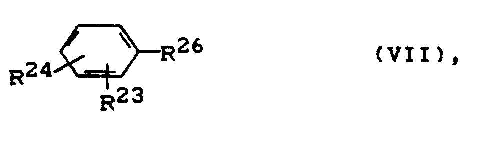
worin
- R²³ und R²⁴: unabhängig voneinander Wasserstoff, Methyl, Ethyl, Fluor oder Chlor bedeuten und R²³ zusätzlich für Hydroxy stehen kann und
- R²⁶: für Wasserstoff, Methyl oder eine der Gruppen 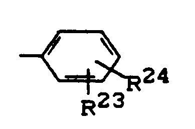 oder 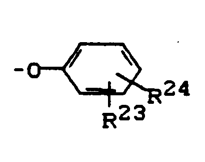 steht.

Ganz besonders bevorzugte Benzol-Verbindungen für das erfindungsgemäße Verfahren sind solche der Formel 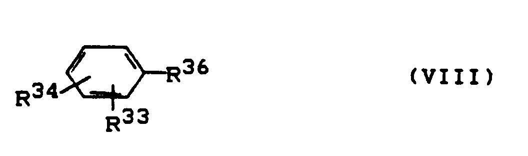
worin
- R³³ und R³⁴: unabhängig voneinander Wasserstoff, Methyl, Ethyl oder Chlor bedeuten und R³³ zusätzlich für Hydroxy stehen kann und
- R³⁶: für Wasserstoff, Methyl oder durch R³³ und R³⁴ substituiertes Phenyl steht.

Weitere bevorzugte Benzole der Formel (II) bzw. (VI) sind solche, in denen einer, bevorzugt zwei, besonders bevorzugt drei der genannten Substituenten für Wasserstoff stehen. In analoger Weise sind weiterhin bevorzugte Benzole solche der Formel (VII) bzw. (III), in denen einer, bevorzugt zwei der genannten Substituenten Wasserstoff bedeuten.

Wichtige Benzol-Verbindungen für das erfindungsgemäße Verfahren sind beispielsweise: Benzol, Toluol, Xylol, Mesitylen, Ethylbenzol, Diethylbenzol, Propylbenzol, Isopropylbenzol, Diisopropylbenzol, Phenol, Kresole, Diphenyl und Diphenylether.

Unter erfindungsgemäß einsetzbarer aktivierter Bleicherde sind Tone zu verstehen, welche einer Säurebehandlung unterworfen wurden. Tone hierfür sind überwiegend Minerale der Montmorillonit-Gruppe, zu der Bentonite, Nontronite, Baydellite und Hectorite gehören. Geeignete Tone für die Herstellung aktivierter Bleicherde finden sich als natürliche Minerale, können aber auch künstlich synthetisiert werden. Aktivierte Bleicherden werden unter vielen Handelsbezeichnungen vertrieben, so beispielsweise als Floridin, Tonsil, Terrana, Clarit, Filtrol, Nordal, Rumsil und anderen. Solche aktivierten Bleicherden sind dem Fachmann bekannt und beispielsweise in Ullmanns Encyclopädie der technischen Chemie, 3. Auflage, Band 4 (1953) S. 541 ff. behandelt.

Die aktivierte Bleicherde wird erfindungsgemäß in einer Menge von 0,05-5 Gew.-%, bevorzugt 0,07-2 Gew.-%, besonders bevorzugt 0,1-1 Gew.-%, bezogen auf den Benzylalkohol bzw. den daraus hervorgegangenen Dibenzylether, eingesetzt.

Das erfindungsgemäße Verfahren wird bei einer Temperatur zwischen 90 und 140°C, bevorzugt bei 95-130°C, besonders bevorzugt bei 100-120°C durchgeführt. In ganz besonders bevorzugter Weise wird bei der Siedetemperatur des Reaktionsgemisches gearbeitet. Dies ist in einigen Fällen der Siedepunkt der einzusetzenden Benzol-Verbindung. Sofern der Siedepunkt der Benzol-Verbindung nicht in den angegebenen Bereich der Reaktionstemperatur fällt, kann der Siedepunkt der Benzol-Verbindung in einer dem Fachmann bekannten Weise durch geringfügige Druckerhöhung (beispielsweise beim Einsatz von Benzol) oder durch entsprechende Druckverminderung (beispielsweise beim Einsatz von Xylol und anderen höhersiedenden Benzol-Verbindungen) erreicht werden. Die Einstellung von Siedebedingungen im Rahmen der genannten Reaktionstemperatur kann jedoch auch durch Zugabe von inerten, d.h. in den Reaktionsablauf nicht eingreifenden Lösungs-bzw. Verdünnungsmitteln erreicht werden. Solche Lösungsmittel haben einen Siedepunkt innerhalb des Bereiches der angegebenen Reaktionstemperatur oder nur wenig darunter. Solche inerten Lösungsmittel sind beispielsweise Cyclohexan, Methyl-cyclohexan, Dimethyl-cyclohexan, Isooctan und andere im Bereich der Reaktionstemperatur siedende (cyclo)aliphatische Kohlenwasserstoffe.

Im Falle des Cyclohexans mit einem Siedepunkt von etwa 81°C stellt sich im Reaktionsgemisch eine Sumpftemperatur ein, die unter dem Einfluß aller Reaktionsteilnehmer höher liegt als die Siedetemperatur des Cyclohexans und zwar umso höher, je weniger Cyclohexan dem Reaktionsgemisch zugesetzt wird. Durch die Menge des zugesetzten inerten Lösungsmittels (beispielsweise des Cyclohexans) kann somit die gewünschte Reaktionstemperatur in gewünschter Weise eingestellt werden.

Im erfindungsgemäßen Verfahren wird Reaktionswasser gebildet; pro umgesetztem Mol Benzylalkohol ist dies 1 Mol Wasser. Das Reaktionswasser kann grundsätzlich im Reaktionsgemisch verbleiben und erst bei der Aufarbeitung abgetrennt werden. In einer bevorzugten Weise wird das Reaktionswasser jedoch bereits während der Kondensationsreaktion destillativ aus dem Reaktionsgemisch entfernt und ausgeschleust. Hierbei wird die Tatsache in vorteilhafter Weise ausgenutzt, daß viele Benzole und inerte Lösungsmittel mit Wasser ein Azeotrop bilden, welches nach der Kondensation außerhalb des Reaktionsgemisches in einem Wasserabscheider wieder in die organische Verbindung und das Wasser getrennt wird. Das Benzol oder das Lösungsmittel oder ein Gemisch von beiden wird wieder in das Reaktionsgemisch zurückgeführt, während das ausgeschleuste Wasser als Maß für den Fortschritt der Reaktion dient.

Die im erfindungsgemäßen Verfahren herstellbaren Benzylbenzole der Formel (I) haben die Struktur 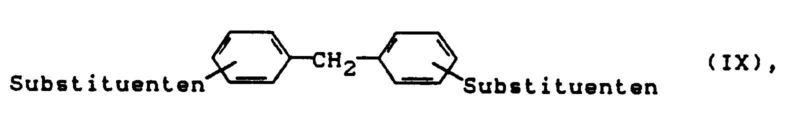
bei der die oben im einzelnen genannten Reste pauschal als "Substituenten" (S) bezeichnet werden.

Das Benzol kann auch zweifach benzyliert werden, so daß sich folgende Struktur ergibt: 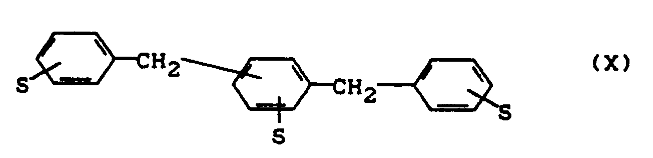
Sofern von einem Phenyl-benzol (=Biphenyl) oder einem Phenoxy-benzol (= Diphenylether) ausgegangen wird (R⁸, R¹⁸, R²⁸ bzw. R³⁸), ergeben sich folgende Strukturen: 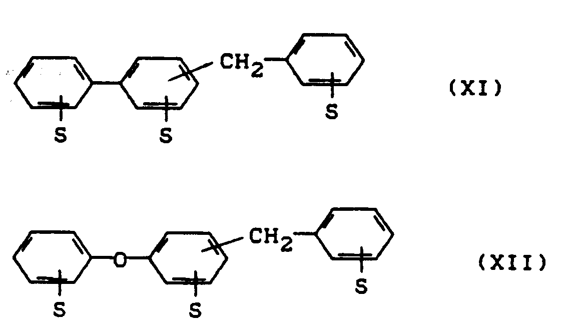
sowie die an einem aromatischen Kern zweifach benzylierten Typen, die an je einem aromatischen Kern einfach benzylierten Typen und Strukturen, die sich vom Tribenzyl-biphenyl oder Tribenzyl-diphenylether ableiten. Schließlich kann eine eingetretene Benzylgruppe ihrerseits benzyliert werden.

Zur Erreichung der verschiedenen Benzylierungsgrade werden 0,5-5 Mol Benzylalkohol pro Mol Benzol eingesetzt. Mit 0,5-1,1 Mol Benzylalkohol erreicht man im wesentlichen eine einfache Benzylierung. Mit 1,5-2,2 Mol Benzylalkohol erreicht man im wesentlichen eine zweifache Benzylierung. Mit noch größeren Mengen erreicht man auf sofort einsehbare Weise weitere erhöhte Benzylierungsgrade. Bei der Einstellung des Benzylierungsgrades 1 treten in untergeordnetem Maße doppelt benzylierte Produkte auf. Bei höheren Benzylierungsgraden wird das Spektrum verschieden hoch benzylierter Produkte breiter. Falls Gemische gewünscht werden, können auch von ganzen Zahlen stärker abweichende Molzahlen angewandt werden, z.B. 1,1-1,5, 1,3-1,9 oder 2,2-2,7 Mol Benzylalkohol pro Mol Benzol oder noch andere (auch höhere) Molbereiche innerhalb des genannten Gesamtbereichs.

Will man auch bei höherer Benzylierung das Spektrum der verschieden hoch benzylierten Benzole eng halten, ist es ferner möglich, das zunächst in der geschilderten Weise einfach benzylierte Benzol zu isolieren und erneut einer Monobenzylierung zu unterwerfen.

Die Produkte des erfindungsgemäßen Verfahrens können destillativ in Einzelkomponenten aufgetrennt werden. Hierbei wird wegen der sehr hohen Siedepunkte mit Vorteil unter vermindertem Druck gearbeitet. Ohne größeren Destillationsaufwand können hierbei die 2-Kern-Verbindungen und die 3-Kern-Verbindungen isoliert werden. Die Isolierung noch höher kondensierter Stoffe wird zunehmend schwieriger, so daß sie mit Vorteil als Gemisch weiter verwendet werden. Die 2-Kern-Verbindungen und 3-Kern-Verbindungen sind nützliche Zwischenprodukte, die einer weiteren Umwandlung nach bekannten Methoden der Aromatenchemie zugänglich sind. Darüber hinaus sind alle erfindungsgemäß herstellbaren Stoffe durch eine hervorragende thermische Beständigkeit ausgezeichnet, so daß sie sich als Wärmeträgermedien eignen. Eine noch weitere hervorragende Eignung ergibt sich durch die hervorragenden Isoliereigenschaften als Elektroisolierflüssigkeiten, beispielsweise für Schaltstationen, Kondensatoren und Transformatoren. Wegen der destillativen Isolierbarkeit bzw. gezielten Herstellung der 2-Kern-und 3-Kern-Verbindungen können diese Verbindungen bereits als Rohprodukte sowohl einzeln als auch als Gemische für die Zwecke als Wärmeträger oder als Elektroisolierflüssigkeit eingesetzt werden. Mit Hilfe des erfingungsgemäßen Verfahrens wird eine große Palette verschiedener Viskositäten und Abstufungen sonstiger Eigenschaften möglich. Die 4-Kern-Verbindungen und noch höhere Verbindungen werden im allgemeinen als Gemisch belassen und finden Verwendung als wertvolle harzartige Massen, beispielsweise zur Einbettung von elektrischen Einrichtungen, als Weichmacher für Polymere, als klebrigmachende Harze in der Gummiindustrie usw.

Für alle genannten Einsätze ist es von ausschlaggebender Bedeutung, daß die Produkte des erfindungsgemäßen Verfahrens absolut chlorfrei sind und damit keine Korrosion oder Selbstzersetzung hervorrufen. Dies ist wichtig im Vergleich etwa zu Benzyl-benzolen, die durch Benzylierung mit Benzylchlorid hergestellt werden; typischerweise hinterbleibt bei der Benzylchlorid-Kondensation nach dem Abdestillieren der niedrigeren Reaktionsprodukte ein teeriger Rückstand, während der harzartige Rückstand des erfindungsgemäßen Verfahrens, dessen Verwendungen soeben genannt wurden, honiggelb ist. Das erfindungsgemäße Verfahren kommt mit einer überraschend niedrigen Menge an aktivierter Bleicherde aus. Das erfindungsgemäße Verfahren hat weiterhin keine Abwasser-und Abluftbelastungen wie die Verfahren des Standes der Technik; insbesondere gibt es keine Formaldehyd-Emission.

Es ist ein wichtiges Kennzeichen des erfindungsgemäßen Verfahrens, daß in Gegenwart eines Verdünnungsmittels gearbeitet wird, die bereits weiter oben zur Einstellung der Reaktionstemperatur unter Siedebedingungen genannt wurden. Diese Verdünnungsmittel dürfen unter den Verfahrensbedingungen nicht benzylierbar sein. Ganz besonders wichtig unter ihnen ist das Cyclohexan.

Das Verdünnungsmittel wird in einer Menge von 40-2000 Gew.-%, bezogen auf das umzusetzende Benzol, eingesetzt. Aus wirtschaftlichen Gründen wird man den oberen Teil des genannten Bereiches nur anwenden, wenn der Benzylierungsgrad sehr genau eingestellt werden soll; ansonsten reichen 40-400 Gew.-%, vielfach bereits 40-200 Gew.-%, bezogen auf das Benzol.

Für den Fall, daß der Benzylierungsgrad 1 angestrebt wird, ist es möglich, an Stelle eines nicht benzylierbaren Verdünnungsmittels das zu benzylierende Benzol im Überschuß als Verdünnung einzusetzen. Dies hat vor allem Bedeutung bei den niedrig siedenden Ausgangsbenzolen, wie Benzol, Toluol, Xylol, Fluorbenzol und Chlorbenzol. Hier werden Verdünnungsgrade im oberen Teil des genannten Bereiches benutzt.

Das erfindungsgemäße Verfahren kann in vielfältiger Weise ausgeführt werden. So ist es beispielsweise in der bereits angegebenen Weise möglich, das entstehende Reaktionswasser erst bei der Aufarbeitung vom Reaktionsgemisch abzutrennen oder in bevorzugter Weise bereits während der Durchführung der Reaktion. Weiterhin ist es möglich, alle Ausgangsmaterialien einschließlich der aktivierten Bleicherde und des Verdünnungsmittels gemeinsam vorzulegen und auf die gewünschte Reaktionstemperatur zu erhitzen. Des weiteren ist es möglich, das Benzol, das Verdünnungsmittel und die aktivierte Bleicherde vorzulegen und auf Reaktionstemperatur zu erhitzen und erst danach den Benzylalkohol zuzugeben. Eine solche Zugabe erfolgt in bevorzugter Weise nach Maßgabe der Wasserentwicklung, wozu in einem solchen bevorzugten Fall das Reaktionswasser in der beschriebenen Weise aus dem Reaktionsgemisch bereits während der Reaktion abgetrennt wird.

Es ist ferner möglich, Lösungsmittel, Benzylalkohol und Bleicherde vorzulegen und unter Ausschleusung von Reaktionswasser eine Eigenkondensation des Benzylalkohols zu starten. Hierbei können sowohl Dibenzylether als auch ein- oder mehrfach benzylierte Benzylether gebildet werden. Nach Zugabe der Benzolverbindung wird die Selbstkondensation abgebrochen und die Benzylgruppen werden auf die Benzol-Verbindung übertragen.

Diese Variante zeigt, daß an Stelle des Benzylalkohols grundsätzlich sein zugehöriger Dibenzylether eingesetzt werden kann. Hierbei wird von der pro Mol Benzylalkohol freiwerdenden molaren Menge Reaktionswasser bereits ein Teil bei der Etherbildung bzw. einer weitergehenden Benzylierung dieses Ethers freigesetzt. Bei der Verfolgung der Benzylierung des Benzols durch die Abspaltung von Reaktionswasser muß dieses vorher bereits abgespaltene Reaktionswasser angerechnet werden. Selbstverständlich wird der gesamte zur Bildung des Dibenzylethers oder der zur Benzylierung eines solchen Dibenzylethers verbrauchte Benzylalkohol als die einzusetzende molare Menge Benzylalkohol angesehen. Diese Bildung des Benzylethers und dessen Aufbrechen ist besonders bei schwach aktiven (z.B. bei halogenierten) Benzolen vorteilhaft.

In einer weiteren vorteilhaften Verfahrensvariante kann das erfindungsgemäße Arbeiten in Gegenwart eines Verdünnungsmittels noch verstärkt werden: So ist es nicht nur möglich, den Benzylalkohol gleichmäßig dosiert (im Laboransatz beispielsweise tropfenweise) auf die Oberfläche des verdünnten Benzols oder besser durch ein Tauchrohr in die Lösung hineinzugeben und dort durch einen intensiven Rührer schnell und gleichmäßig zu verteilen, sondern es kann auch ein Verdünnungsmittel als Vehikel für den einzudosierenden Benzylalkohol verwendet werden. Ein solches Vehikel ist beispielsweise das aus dem Wasserabscheider zurücklaufende, ursprünglich für die Benzolverdünnung eingesetzte Verdünnungsmittel oder ein Fördergas, z.B. Luft oder N₂, mit denen der Benzylalkohol neben dem vorgelegten Benzol ebenfalls verdünnt wird. Insbesondere ein solches Fördergas ist vorteilhaft, weil damit der Benzylalkohol aus einem Tauchrohr in der Nähe des Rührers in die verdünnte Lösung des Benzols eingedüst wird.

Zur Aufarbeitung des Reaktionsproduktes wird das Verdünnungsmittel vor oder nach der Abtrennung der Bleicherde abdestilliert und das benzylierte Benzol fraktioniert oder als anfallendes Gemisch verwendet. Bei der Herstellung höher benzylierter und damit höher viskoser Reaktionsprodukte kann der Fall eintreten, daß die Lösekraft des Verdünnungsmittels für eine störungsfreie Aufarbeitung nicht ausreichend ist, da das Verdünnungsmittel nicht benzylierbar sein darf und daher aliphatisch ist. Hier hilft es, wenn nach dem Verbrauch des gesamten eingesetzten Benzylalkohols vor oder während des Abdestillierens des Verdünnungsmittels ein aromatisches Hilfslösungsmittel, beispielsweise Benzol oder Toluol, zugesetzt und dann weiter wie beschrieben verfahren wird.

### Beispiele

### Beispiel 1 (Benzyltoluol)

3.170 ml Toluol und 10,5 g Bleicherde (Tonsil K 10 der Fa. Südchemie AG) wurden in einem Rührkessel mit Destillationsübergang, Kühler und Wasserabscheider zum Sieden erhitzt. In ca. 2 Stunden wurden dann 216 g Benzylalkohol eingedüst, d.h. über ein eintauchendes dünnes Rohr wurde unter Stickstoffströmung (100 l/h) Benzylalkohol eingetropft und dadurch schnell und gleichmäßig verteilt und zur Reaktion gebracht.

Die Zudosierung des Benzylalkohols konnte zügig erfolgen und wurde dem Wasseranfall entsprechend äquivalent gehalten.

| Zeit (h.min.) | Sumpftemp. °C | Benzylalkohol (ml) | abdestill. Wasser (ml) |
|---|---|---|---|
| 0 | 110 | 0 | |
| 0.30 | 110 | 60 | 9 |
| 1.00 | 111 | 110 | 20 |
| 1.30 | 112 | 170 | 29 |
| 1.50 | 112 | 210 | 34 |
| ca. 1 - 2 g Wasser gingen mit dem Stickstoffstrom verloren. | | | |

Nach beendeter Kondensation genügte eine Nachreaktionszeit von ca. 30 Minuten. Anschließend wurde das nicht umgesetzte Toluol über eine kurze Kolonne abdestilliert und damit gleichzeitig für den Wiedereinsatz gereinigt. In einem Rotationsverdampfer wurde bei 20 - 50 mm Hg und einer Sumpftemperatur von max. 100°C das restliche Toluol aus dem Rohkondensat entfernt und ebenfalls wiedergewonnen. Zum Schluß wurde die Bleicherde durch Filtration entfernt.

Im Mittel mehrerer Versuche wurden 345 g = 95 % der theoretischen Ausbeute (bezogen auf Benzylalkohol) an Benzyltoluol erhalten. Das Produkt enthielt nur wenige Prozente Dibenzyltoluol (6 - 8 % in mehreren Versuchsläufen); es war in der anfallenden Form als wasserklare Flüssigkeit unmittelbar einsetzbar, z.B. als Elektroisolierflüssigkeit. Chlor war nicht nachweisbar.

### Beispiel 2 (Polybenzyldiphenyl)

Bei der Synthese höherkondensierter Produkte wurde die Eintragung des Benzylalkohols bei einer stets konstanten Kondensationstemperatur vorgenommen, die im günstigen Fall bei 108 - 110°C lag. Die Eintragung des Benzylalkohols erfolgte in gleicher Weise wie in Beispiel 1. Die erforderliche konstante Kondensationstemperatur von 108-110°C erreichte man durch Zugabe von Cyclohexan als einem für die Reaktion inerten Wasserschlepper. Dabei setzte man dem vorgelegten Diphenyl gerade so viel Cyclohexan zu, daß aus dem Cyclohexan-Diphenyl-Gemisch das Cyclohexan bei einer Sumpftemperatur von 108 - 110°C am Rückfluß siedete. Durch weitere gleichmäßige Zudosierung von Cyclohexan während des ganzen Kondensationsprozesses wurde die Sumpftemperatur und damit die Kondensationstemperatur bei 108 - 110°C konstant gehalten.

Nach beendeter Kondensation erhielt man viskose Reaktionsgemische. Daraus war die Abtrennung der Bleicherde nicht direkt möglich, gelang aber durch Zentrifugieren nach Verdünnung mit zusätzlichem Cyclohexan im Verhältnis 1:1.

In einer weiteren Aufarbeitungsvariante wurde nach völligem Umsatz des Benzylalkohols Xylol oder Toluol zugesetzt. Bei kontinuierlicher Xylol- bzw. Toluol-Zugabe wurde das bei der Kondensation verwandte Cyclohexan abdestilliert und für den erneuten Einsatz wiedergewonnen. War das Kondensat in einer ausreichenden Menge Xylol bzw. Toluol gelöst, konnte die Bleicherde aus der Cyclohexan-freien Lösung durch Zentrifugieren entfernt werden. Aus dem nun von Bleicherde befreiten Endprodukt wurde das Lösungsmittel (Xylol bzw. Toluol) durch Destillation entfernt und wiedergewonnen. Als Sumpf blieb das lösungsmittelfreie Kondensationsprodukt zurück.

Im einzelnen wurde wie folgt gearbeitet:
Zu 3 Mol = 462 g Diphenyl wurden 300 ml Cyclohexan zugesetzt und bei Rückfluß die Sumpftemperatur auf 108 - 110°C eingestellt. Danach wurden 4 g Bleicherde zugegeben und anschließend 7,5 Mol = 810 g Benzylalkohol mit Stickstoff (100 l/h) über ein Tauchrohr eingeblasen.

| Zeit (h.min.) | Sumpftemp. °C | Benzylalkohol (ml) | abdestill. Wasser (ml) | Cyclohexanzusatz (ml) |
|---|---|---|---|---|
| 0 | 108 | 0 | 0 | |
| 0.20 | 110 | 100 | 10 | |
| 0.50 | 110 | 200 | 27 | 100 |
| 1.20 | 110 | 300 | 43 | |
| 1.40 | 110 | 400 | 58 | 200 |
| 2.10 | 110 | 500 | 75 | |
| 2.40 | 110 | 600 | 91 | 300 |
| 3.15 | 110 | 700 | 108 | |
| 3.45 | 110 | 800 | 115 | 400 |
| 4.45 | 110 | 810 | 125*⁾ | |
| | | | | S̅a̅.̅ 4̅0̅0̅ |

| | | | | |
|---|---|---|---|---|
| *⁾ Theoretisch waren 135 g H₂O zu erwarten. Durch die N₂-Strömung ging Wasser verloren. | | | | |

Im Rotationsverdampfer wurde das Cyclohexan abdestilliert. Man erhielt 1.156 g Rohkondensat mit Bleicherde und minimaler Cyclohexanrestfeuchte (theoretisch 1.133 g).

Das Rohkondensat wurde mit Toluol (1:1) verdünnt und zentrifugiert. Im Rotationsverdampfer wurde anschließend das Toluol abdestilliert.

### Beispiel 3 (Tribenzyltoluol)

Nach der Vorschrift des Beispiels 2 wurde im einzelnen wie folgt gearbeitet:
Zu 600 g Benzyltoluol wurden 300 ml Cyclohexan zugesetzt und bei Rückfluß die Sumpftemperatur auf 110°C eingestellt. Danach wurden 4 g Tonsil K 10 zugegeben und anschließend 800 g Benzylalkohol mit Stickstoff (100 l/h) über ein Tauchrohr eingeblasen.

| Zeit (h.min.) | Sumpftemp. °C | Benzylalkohol (ml) | H₂O-Anfall (ml) | Cyclohexanzusatz (ml) |
|---|---|---|---|---|
| 0 | 107 | 0 | 0 | |
| 0.30 | 110 | 100 | 15 | |
| 1.00 | 110 | 200 | 28 | 100 |
| 1.30 | 110 | 300 | 45 | |
| 2.00 | 110 | 400 | 58 | 200 |
| 2.30 | 110 | 500 | 75 | |
| 3.00 | 110 | 600 | 90 | 300 |
| 3.35 | 110 | 700 | 108 | |
| 4.10 | 110 | 800 | 122 | 400 |
| 4.40 | 110 | - | 122*⁾ | 500 |

| | | | | |
|---|---|---|---|---|
| *⁾ Theoretisch waren 133 g H₂O zu erwarten. Durch die N₂-Strömung ging Wasser verloren. | | | | |

Das Reaktionsgemisch wurde mit 1,5 l Toluol versetzt und anschließend zentrifugiert.

Das Rohkondensat wurde nach Abdestillieren des Lösungsmittels gewonnen.

### Beispiel 4

In einem Reaktor mit Rührer und Wasserabscheider wurden 460 g frisches Toluol und 1719 g zurückgeführtes Toluol gemeinsam mit 2 g Tonsil K 10 zum Sieden erhitzt. In die siedende Reaktionsmischung wurden 650 g Benzylalkohol nach Maßgabe der Wasserentwicklung eingetropft. Das Wasser wurde als Azeotrop mit Toluol abdestilliert und in einem Wasserabscheider vom Toluol getrennt. Das Toluol wurde dem Reaktionsansatz wieder zugesetzt. Insgesamt entwickelten sich 108 g Wasser. Nach Beendigung der Reaktion (erkennbar am Aufhören der Wasserentwicklung) wurden 3 g Calciumoxid zum Reaktionsansatz gegeben und verrührt. Anschließend wurde filtriert und das Toluol aus dem Filtrat abdestilliert (Rückführung von 1719 g Toluol). Man erhielt eine Rohausbeute von 937 g, die einer Vakuumdestillation unterworfen wurden. Hierbei erhielt man eine erste Fraktion, bestehend aus Benzyltoluol, in einer Menge von 687 g und eine zweite Fraktion, bestehend aus Dibenzyl-toluol, in einer Menge von 172 g. Die Fraktionen 1 und 2 in einer Gesamtmenge von 859 g stellen 91,6 % der Rohausbeute dar. Die Vakuumdestillation ergab weiterhin 67 g = 7,1 % der Rohausbeute an honiggelbem Harz. Der Rest waren Handhabungsverluste.

## Patentansprüche

1. Verfahren zur Herstellung von Benzyl-benzolen der Formel 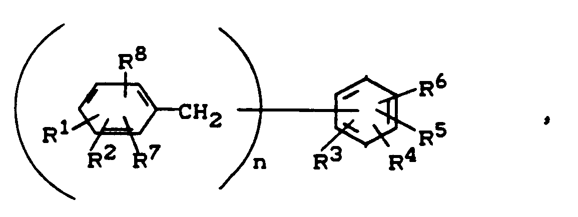 in der
R¹, R², R³, R⁴ und R⁵ unabhängig voneinander Wasserstoff, C₁-C₄-Alkyl, C₁-C₄-Alkoxy, Fluor oder Chlor bedeuten, wobei R¹, R², R³ und R⁴ unabhängig voneinander auch für Hydroxy stehen können und R⁵ zusätzlich C₅-C₈-Alkyl sein kann,
R⁶ für Wasserstoff oder eine der Gruppen 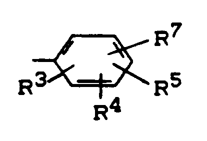 oder 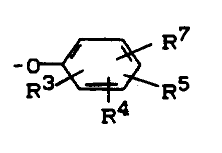 steht,
R⁷ für Wasserstoff oder die Gruppe 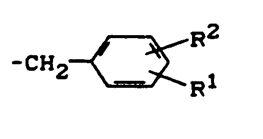 steht und
n die Zahl 1 oder 2 darstellt und für den Fall, daß einer der Reste R³, R⁴, R⁵ oder R⁶ Wasserstoff bedeutet, auch die Zahl 3 darstellen kann,
dadurch gekennzeichnet, daß man ein Benzol der Formel 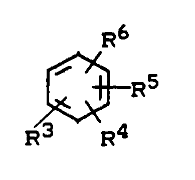 mit einem Benzylalkohol der Formel 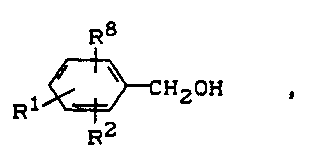 wobei R¹ bis R⁵ die oben angegebene Bedeutung haben,
R⁶ für Wasserstoff oder eine der Gruppen 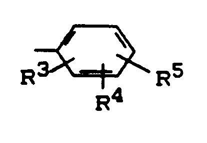 oder 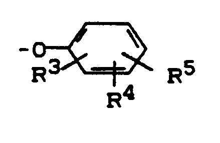 steht und
R⁸ Wasserstoff oder Hydroxymethyl bedeutet,
in einem Verhältnis von 0,4 bis 15 Mol Benzol zu 1 Mol Benzylalkohol in Gegenwart einer Menge an aktivierter Bleicherde von 0,05-5 Gew.-%, bezogen auf die Menge an Benzylalkohol, bei einer Temperatur zwischen 90°C und 140°C in Gegenwart eines Verdünnungsmittels umsetzt.

2. Verfahren nach Anspruch 1, dadurch gekennzeichnet, daß Benzylalkohole der Formel 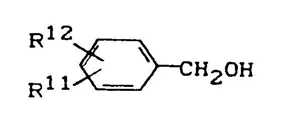 eingesetzt werden, worin
R¹¹ und R¹² unabhängig voneinander Wasserstoff, Methyl, Ethyl, Fluor oder Chlor bedeuten und R¹¹ zusätzlich für Hydroxymethyl stehen kann,

3. Verfahren nach Anspruch 2, dadurch gekennzeichnet, daß Benzylalkohole der Formel 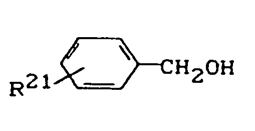 eingesetzt werden, worin
R²¹ Wasserstoff, Methyl oder Ethyl bedeutet.

4. Verfahren nach Anapruch 1, dadurch gekennzeichnet, daß Benzole der Formel 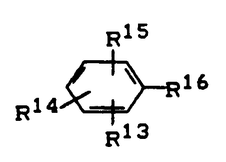 eingesetzt werden, worin
R¹³, R¹⁴ und R¹⁵ unabhängig voneinander Wasserstoff, C₁-C₄-Alkyl, Fluor oder Chlor bedeuten und R¹³ weiterhin für Hydroxy stehen kann und
R¹⁶ für Wasserstoff oder eine der Gruppen 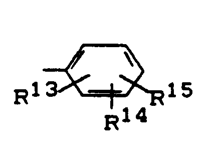 oder 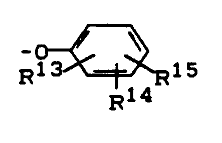 steht.

5. Verfahren nach Anspruch 4, dadurch gekennzeichnet, daß Benzole der Formel 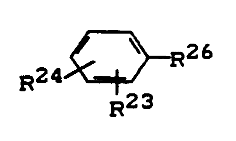 eingesetzt werden, worin
R²³ und R²⁴ unabhängig voneinander Wasserstoff, Methyl, Ethyl, Fluor oder Chlor bedeuten und R²³ zusätzlich für Hydroxy stehen kann und
R²⁶ für Wasserstoff, Methyl oder eine der Gruppen 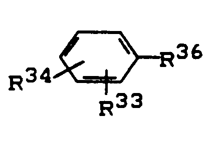 oder 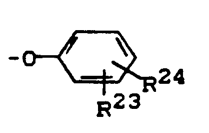 steht.

6. Verfahren nach Anspruch 1, dadurch gekennzeichnet, daß Benzole der Formel 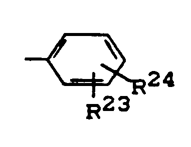 eingesetzt werden, worin
R³³ und R³⁴ unabhängig voneinander Wasserstoff, Methyl, Ethyl oder Chlor bedeuten und R³³ zusätzlich für Hydroxy stehen kann und
R³⁶ für Wasserstoff, Methyl oder durch R³³ und R³⁴substituiertes Phenyl steht.

7. Verfahren nach Anspruch 1, dadurch gekennzeichnet, daß die aktivierte Bleicherde in einer Menge von 0,07-2 Gew.-%, bevorzugt 0,1-1 Gew.-%, bezogen auf den Benzylalkohol, eingesetzt wird.

8. Verfahren nach Anspruch 1, dadurch gekennzeichnet, daß als Verdünnungsmittel Cyclohexan, Methyl-cyclohexan oder Dimethyl-cyclohexan in einer Menge von 40-2000 Gew.-%, bezogen auf das zu benzylierende Benzol, eingesetzt werden.

9. Verfahren nach Anspruch 1, dadurch gekennzeichnet, daß der Benzylalkohol in einer durch das Verdünnungsmittel oder durch ein Fördergas verdünnten Form eingesetzt wird.

10. Verfahren nach Anspruch 9, dadurch gekennzeichnet, daß der Benzylalkohol mit Hilfe eines Luft- oder N₂-Stromes über ein Tauchrohr in das verdünnte Benzol eingedüst wird.

## Claims

1. Process for the preparation of benzylbenzenes of the formula 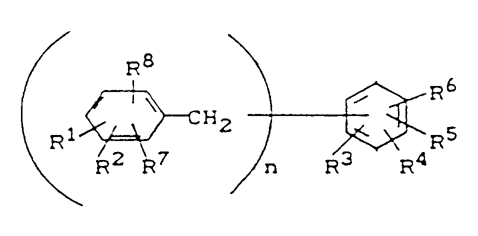 in which
R¹, R², R³, R⁴ and R⁵, independently of one another, denote hydrogen, C₁-C₄-alkyl, C₁-C₄-alkoxy, fluorine or chlorine, it also being possible for R¹, R², R³ and R⁴ to represent, independently of one another, hydroxyl and for R⁵ also to be C₅-C₈-alkyl,
R⁶ represents hydrogen or one of the groups 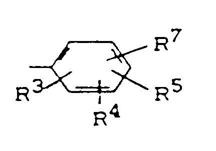 or 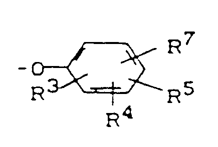
R⁷ represents hydrogen or the group 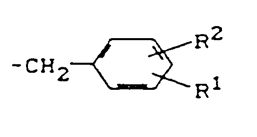 and
n represents the number 1 or 2 and, in the case where one of the radicals R³, R⁴, R⁵ or R⁶ denotes hydrogen, can also represent the number 3,
characterised in that a benzene of the formula 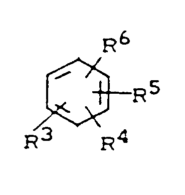 is reacted with a benzyl alcohol of the formula 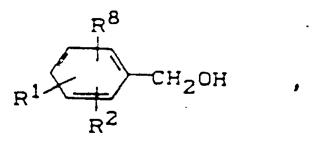 R¹ to R⁵ having the abovementioned meaning,
R⁶ representing hydrogen or one of the groups 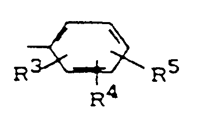 or 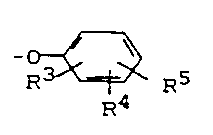 and
R⁸ denoting hydrogen or hydroxymethyl,
in a ratio of 0.4 to 15 mol of benzene to 1 mol of benzyl alcohol in the presence of activated bleaching earth in an amount of 0.05 to 5 % by weight, relative to the amount of benzyl alcohol, at a temperature of between 90°C and 140°C in the presence of a diluent.

2. Process according to Claim 1, characterised in that the benzyl alcohols used are those of the formula 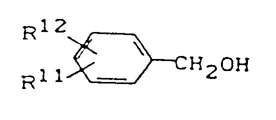 in which
R¹¹ and R¹², independently of one another, denote hydrogen, methyl, ethyl, fluorine or chlorine and R¹¹ can additionally represent hydroxymethyl.

3. Process according to Claim 2, characterised in that the benzyl alcohols used are those of the formula 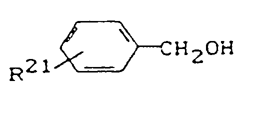 in which
R²¹ denotes hydrogen, methyl or ethyl.

4. Process according to Claim 1, characterised in that the benzenes used are those of the formula 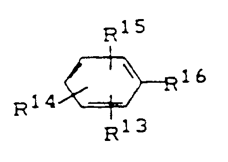 in which
R¹³, R¹⁴ and R¹⁵, independently of one another, denote hydrogen, C₁-C₄-alkyl, fluorine or chlorine and R¹³ can furthermore represent hydroxyl, and
R¹⁶ represents hydrogen or one of the groups 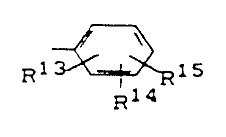 or

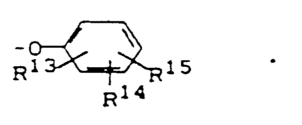

5. Process according to Claim 4, characterised in that the benzenes used are those of the formula 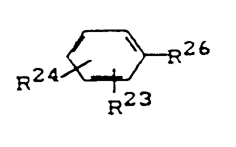 in which
R²³ and R²⁴, independently of one another, denote hydrogen, methyl, ethyl, fluorine or chlorine and R²³ can additionally represent hydroxyl, and
R²⁶ represents hydrogen, methyl or one of the groups 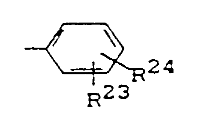 or

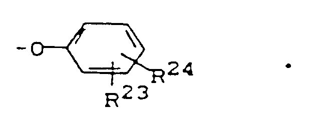

6. Process according to Claim 1, characterised in that the benzenes used are those of the formula 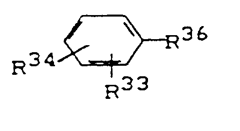 in which
R³³ and R³⁴, independently of one another, denote hydrogen, methyl, ethyl or chlorine and R³³ can additionally represent hydroxyl, and
R³⁶ represents hydrogen, methyl or R³³- and R³⁴-substituted phenyl.

7. Process according to Claim 1, characterised in that the activated bleaching earth is used in an amount of 0.07 - 2 % by weight, preferably 0.1 - 1 % by weight, relative to the benzyl alcohol.

8. Process according to Claim 1, characterised in that cyclohexane, methylcyclohexane and dimethylcyclohexane are used in an amount of 40-2000 % by weight, relative to the benzene to be benzylated, as the diluent.

9. Process according to Claim 1, characterised in that the benzyl alcohol is used diluted by the diluent or by a carrier gas.

10. Process according to Claim 9, characterised in that the benzyl alcohol is carried into the diluted benzene through a dip tube by an air or N₂ stream.

## Revendications

1. Procédé pour produire des benzylbenzènes de formule : 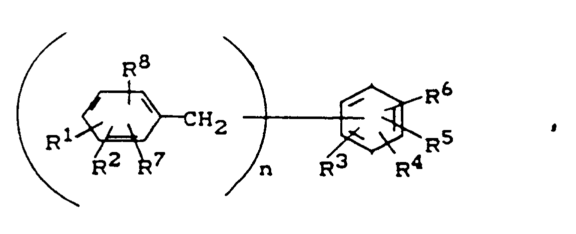 dans laquelle
R¹, R², R³, R⁴ et R⁵ représentent chacun, indépendamment l'un de l'autre, un atome d'hydrogène, un groupe alkyle en C₁-C₄, alcoxy en C₁-C₄, un atome de fluor ou de chlore, les symboles R¹, R², R³ et R⁴ pouvant également , indépendamment l'un de l'autre, représenter chacun un groupe hydroxyle et R⁵pouvant représenter en outre un groupe alkyle en C₅-C₈,
R⁶ représente un atome d'hydrogène ou l'un des groupes 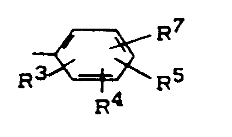 ou 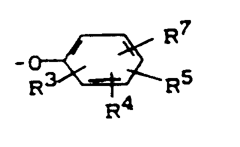 R⁷ représente un atome d'hydrogène ou le groupe 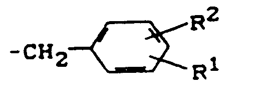 n est un nombre valant 1 ou 2 et, si l'un des restes R³, R⁴, R⁵ ou R⁶ représente un atome d'hydrogène, n peut également valoir 3,
procédé caractérisé en ce qu'on fait réagir un benzène de formule : 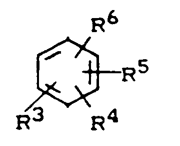 avec un alcool benzylique de formule : 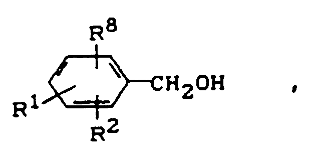 les symboles R¹ à R⁵ ayant le sens indiqué ci-dessus,
R⁶ représentant un atome d'hydrogène, ou l'un des groupes 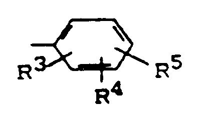 ou 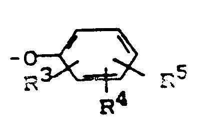 et,
R⁸ représentant un atome d'hydrogène ou un groupe hydroxyméthyle,
la réaction étant réalisée selon un rapport de 0,4 à 15 mol de benzène pour 1 mol d'alcool benzylique, en présence d'une quantité de terre décolorante activée représentant 0,05% à 5%, par rapport à la quantité de l'alcool benzylique, à une température comprise entre 90°C et 140°C en présence d'un diluant.

2. Procédé selon la revendication 1, caractérisé en ce qu'on utilise des alcools benzyliques de formule : 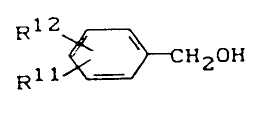 dans laquelle
R¹¹ et R¹² représentent chacun, indépendamment l'un de l'autre, un atome d'hydrogène, un groupe méthyle, éthyle, un atome de fluor ou de chlore, R¹¹ pouvant représenter en outre un groupe hydroxyméthyle.

3. Procédé selon la revendication 2, caractérisé en ce qu'on utilise des alcools benzyliques de formule : 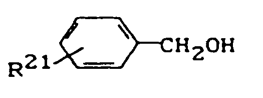 dans laquelle
R²¹ représente un atome d'hydrogène ou un groupe méthyle ou éthyle.

4. Procédé selon la revendication 1, caractérisé en ce qu'on utilise des benzènes de formule : 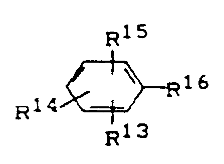 dans laquelle
R¹³, R¹⁴ et R¹⁵ représentent chacun, indépendamment l'un de l'autre, un atome d'hydrogène, un groupe alkyle en C₁-C₄, un atome de fluor ou de chlore, et R¹³ peut représenter en outre un groupe hydroxyle, et
R¹⁶ représente un atome d'hydrogène ou l'un des groupes 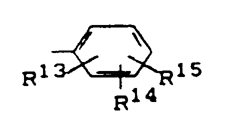 ou

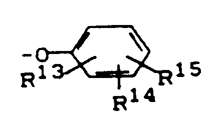

5. Procédé selon la revendication 4, caractérisé en ce qu'on utilise des benzènes de formule : 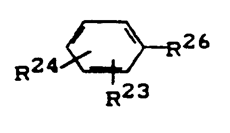 dans laquelle
R²³ et R²⁴ représentent chacun, indépendamment l'un de l'autre, un atome d'hydrogène, un groupe méthyle, éthyle, un atome de fluor ou de chlore et R²³ peut représenter en outre un groupe hydroxyle,
R²⁶ peut représenter un atome d'hydrogène, un groupe méthyle ou l'un des groupes 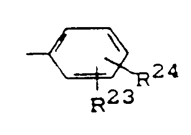 ou

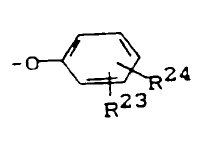

6. Procédé selon la revendication 1, caractérisé en ce qu'on utilise des benzènes de formule : 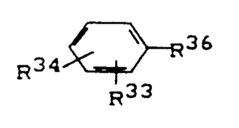 dans laquelle
R³³ et R³⁴ représentent chacun, indépendamment l'un de l'autre, un atome d'hydrogène, un groupe méthyle, éthyle ou chloro, et R³³ peut représenter en outre un groupe hydroxyle, et
R³⁶ représente un atome d'hydrogène, un groupe méthyle ou bien un groupe phényle substitué par R³³ et R³⁴.

7. Procédé selon la revendication 1, caractérisé en ce qu'on utilise une quantité de la terre décolorante activée représentant 0,07 à 2% en poids, de préférence 0,1 à 1% en poids, par rapport à l'alcool benzylique.

8. Procédé selon la revendication 1, caractérisé en ce qu'on utilise comme diluant le cyclohexane, le méthylcyclohexane ou le diméthylcyclohexane, en une quantité de 40 à 2 000% en poids, par rapport au benzène à benzyler.

9. Procédé selon la revendication 1, caractérisé en ce qu'on utilise l'alcool benzylique sous une forme diluée à l'aide du diluant ou à l'aide d'un véhicule gazeux.

10. Procédé selon la revendication 9, caractérisé en ce qu'on insuffle l'alcool benzylique à l'aide d'un courant d'air ou de N₂, par l'intermédiaire d'un tube plongeur, dans le benzène dilué.
